# EUROPEAN PATENT APPLICATION

(11) **EP 1 491 551 A1**
(43) Date of publication of application: **29.12.2004**
(21) Application number: 03715734.4
(22) Date of filing: 02.04.2003
(51) Int. Cl.: C07J 1/00, A61K 31/566, A61P 19/10, A61P 35/00, A61P 43/00

(54) **ESTRONE DERIVATIVE AND PROCESS FOR PRODUCING THE SAME**

(30) Priority: 02.04.2002 JP 2002100158
(71) Applicant: CHUGAI SEIYAKU KABUSHIKI KAISHA, Tokyo, 115-8543 (JP)
(72) Inventor: Nabuchi, Yoshiaki, Gotenba-shi, Shizuoka 412-8513 (JP); Araya, Hiroshi, Gotenba-shi, Shizuoka 412-8513 (JP); Kawata, Setsu, Gotenba-shi, Shizuoka 412-8513 (JP); Morikawa, Kazumi, Gotenba-shi, Shizuoka 412-8513 (JP); Kanbe, Yoshitake, Gotenba-shi, Shizuoka 412-8513 (JP); Ohtake, Yoshihito, Gotenba-shi, Shizuoka 412-8513 (JP); Kaiho, Shinichi, Gotenba-shi, Shizuoka 412-8513 (JP); Taniguchi, Kenji, Gotenba-shi, Shizuoka 412-8513 (JP); Tsunenari, Toshiaki, Gotenba-shi, Shizuoka 412-8513 (JP); Takasu, Hisashi, Gotenba-shi, Shizuoka 412-8513 (JP)
(74) Representative: VOSSIUS & PARTNER
(86) International application number: PCT/JP2003/004222
(87) International publication number: WO 2003/087120

(57) **Abstract**

A compound of formula (I) 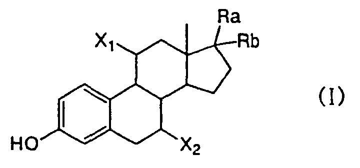 (where X₁ and X₂ represent independently a hydrogen atom or a group of formula (II) 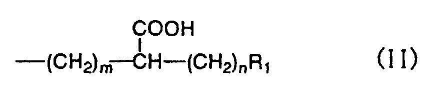 R₁ represents a linear or branched halogenoalkyl group having 1-7 carbon atoms; Ra represents a hydroxyl group and Rb represents a linear or branched alkynyl group having 2-5 carbon atoms, or Ra and Rb, when taken together with the carbon to which they are bound, represent a carbonyl group; m is an integer of 2-14; n is an integer of 2-7; provided that X₁ and X₂ are not both a hydrogen atom), stereoisomers of the compound, or hydrates, salts or esters thereof, as well as a pharmaceutical composition containing a therapeutically effective amount of the compound.

## Description

### TECHNICAL FIELD

This invention relates to novel estrone derivatives, processes for producing them and pharmaceuticals containing said derivatives.

### BACKGROUND ART

Bones are known as dynamic organs that are constantly in the processes of bone resorption and bone formation. Usually, bone resorption by osteoclasts and bone formation by osteoblasts are in balance to control the bone mass constant. However, once the balance is upset and bone resorption predominates bone formation for a certain period of time, osteoporosis is believed to manifest itself.

Osteoclasts are multinucleated cells, that originate from hematopoietic cells of monocyte/macrophage family. Osteoclast precursor cells differentiate into osteoclasts upon stimulation with the osteoclast differentiation factor RANKL (receptor activator of NF-κB ligand) and the M-CSF secreted from osteoblasts. It is known that RANKL is expressed on the membranes of osteoblasts by the bone resorption factor and promotes the differentiation into osteoclasts through signal transmission to osteoclast precursor cells mediated by the RANK (receptor activator of NF-κB) which is a receptor expressed on the membranes of osteoclast precursor cells (see, for example, Proc. Natl. Acad. Sci. USA, 95, 3597-3602, 1998; Jikken Igaku, Vol. 16, No. 11, 1372-1379, 1998; Nippon Rinsho, Vol. 60, Extra Issue 3, 679-687, 2002; Biochem. Biophys. Res. Commun. 253, 395-400, 1998).

The effect of suppressing the function of RANKL has been studied by using OPG (osteoprotegerin) which is a molecule that suppresses the differentiation of osteoclast precursor cells into osteoclasts. OPG is a decoy receptor for RANKL and known to suppress the differentiation of osteoclast precursor cells into osteoclasts by interfering with the function of RANKL (see, for example, Nippon Rinsho, Vol. 60, Extra Issue 3, 679-687, 2002). For example, observations that have been reported to date include: dose-dependent increase of bone density and bone mass resulting from the administration of OPG into normal rats (Endocrinology, 139, 1329-1337, 1998); a decrease in osteoclast numbers in mice overexpressing OPG and an increase in bone density in the long bones and vertebrae in mice overexpressing OPG (Cell, 89, 309-319, 1997); the development of abnormalities typical of osteoporosis in OPG gene deficient model mice (Biochem. Biophys. Res. Commun., 247, 610-615, 1998; Genes. Dev., 12, 1260-1268, 1998); and a decrease in osteoclast numbers and an increase in bone mass resulting from the administration of OPG (its derivatives) to ovariectomized model rats (Cell, 89, 309-319, 1997; Nippon Rinsho, Vol. 60, Extra Issue 3, 679-687, 2002).

Therefore, it is anticipated that if the differentiation of osteoclast precursor cells into osteoclasts can be suppressed by interfering with the function of RANKL, bone resorption can be suppressed or bone mass can be increased, leading to the development of effective preventives or therapeutics of osteoporosis.

### DISCLOSURE OF THE INVENTION

The present invention aims at providing estrone derivatives useful as pharmaceuticals.

The present inventors conducted intensive studies with a view to solving the aforementioned problems of the prior art and, as a result, they found that estrone derivatives of formula (I) were useful as pharmaceuticals and completed the present invention.

In brief, the present invention provides a compound of formula (I) 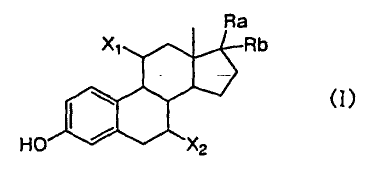 (where X₁ and X₂ represent independently a hydrogen atom or a group of formula (II) 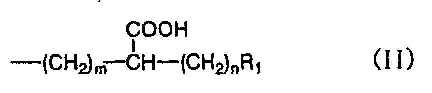 R₁ represents a linear or branched halogenoalkyl group having 1-7 carbon atoms;
Ra represents a hydroxyl group and Rb represents a linear or branched alkynyl group having 2-5 carbon atoms, or Ra and Rb, when taken together with the carbon to which they are bound, represent a carbonyl group;
m is an integer of 2-14;
n is an integer of 2-7;
provided that X₁ and X₂ are not both a hydrogen atom), stereoisomers of the compound, or hydrates, salts or esters thereof.

The invention also provides a compound of formula (Ia) 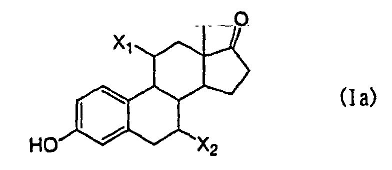 (where X₁ and X₂ represent independently a hydrogen atom or a group of formula (II) 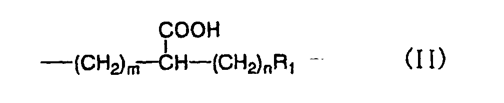 R₁ represents a linear or branched halogenoalkyl group having 1-7 carbon atoms;
m is an integer of 2-14;
n is an integer of 2-7;
provided that X₁ and X₂ are not both a hydrogen atom), stereoisomers of the compound, or hydrates, salts or esters thereof.

The invention further provides a compound of formula (Ib) 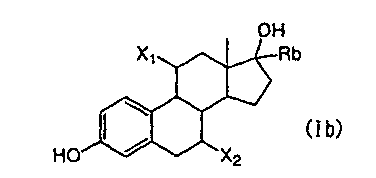 (where X₁ and X₂ represent independently a hydrogen atom or a group of formula (II) 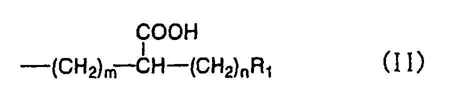 R₁ represents a linear or branched halogenoalkyl group having 1-7 carbon atoms;
Rb represents a linear or branched alkynyl group having 2-5 carbon atoms;
m is an integer of 2-14;
n is an integer of 2-7;
provided that X₁ and X₂ are not both a hydrogen atom), stereoisomers of the compound, or hydrates, salts or esters thereof.

In another aspect, the present invention provides pharmaceutical compositions comprising therapeutically effective amounts of the compounds of formula (I), stereoisomers of the compounds, or hydrates, salts or esters thereof as an active ingredient. While the pharmaceutical compositions are not limited to any particular indications, they may be employed to prevent or treat osteoporosis or breast cancer. The compounds of formula (I), stereoisomers of the compounds, or hydrates, salts or esters thereof can be employed to produce pharmaceuticals comprising therapeutically effective amounts of said compounds. While said pharmaceuticals are not limited to any particular indications, they may be employed to prevent or treat osteoporosis or breast cancer.

In yet another aspect, the present invention provides a process for producing a compound of formula (Ia) 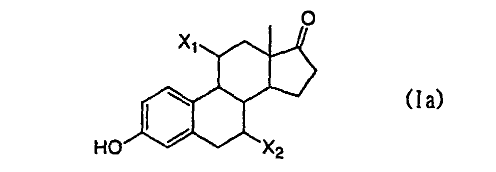 (where X₁ and X₂ represent independently a hydrogen atom or a group of formula (II) 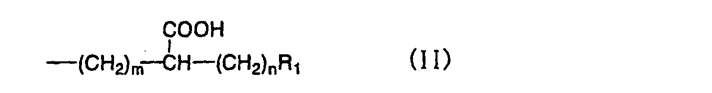 R₁ represents a linear or branched halogenoalkyl group having 1-7 carbon atoms;
m is an integer of 2-14;
n is an integer of 2-7;
provided that X₁ and X₂ are not both a hydrogen atom), stereoisomers of the compound, or hydrates, salts or esters thereof, said process including the step of oxidizing a compound of formula (III) 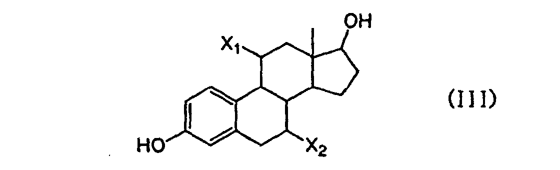 (where X₁ and X₂ represent independently a hydrogen atom or a group of formula (II) 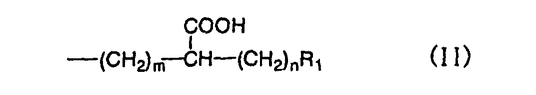 R₁ represents a linear or branched halogenoalkyl group having 1-7 carbon atoms;
m is an integer of 2-14;
n is an integer of 2-7;
provided that X₁ and X₂ are not both a hydrogen atom), stereoisomers of the compound, or hydrates, salts or esters thereof.

Further in addition, the present invention provides a process for producing a compound of formula (Ib) 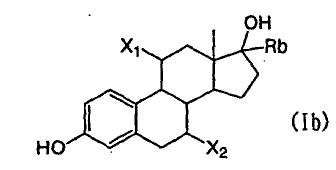 (where X₁ and X₂ represent independently a hydrogen atom or a group of formula (II) 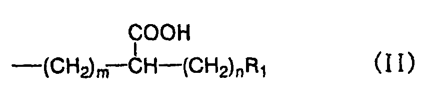 R₁ represents a linear or branched halogenoalkyl group having 1-7 carbon atoms;
Rb represents a linear or branched alkynyl group having 2-5 carbon atoms;
m is an integer of 2-14;
n is an integer of 2-7;
provided that X₁ and X₂ are not both a hydrogen atom), stereoisomers of the compound, or hydrates, salts or esters thereof, said process including the step of alkynylating a compound of formula (Ia) 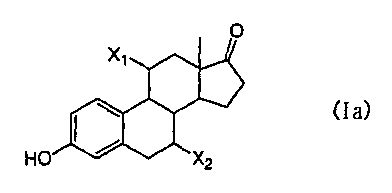 (where X₁ and X₂ represent independently a hydrogen atom or a group of formula (II) 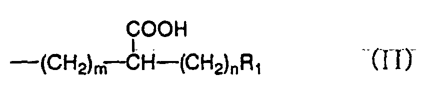 R₁ represents a linear or branched halogenoalkyl group having 1-7 carbon atoms;
m is an integer of 2-14;
n is an integer of 2-7;
provided that X₁ and X₂ are not both a hydrogen atom), stereoisomers of the compound, or hydrates, salts or esters thereof.

### BRIEF DESCRIPTION OF THE DRAWING

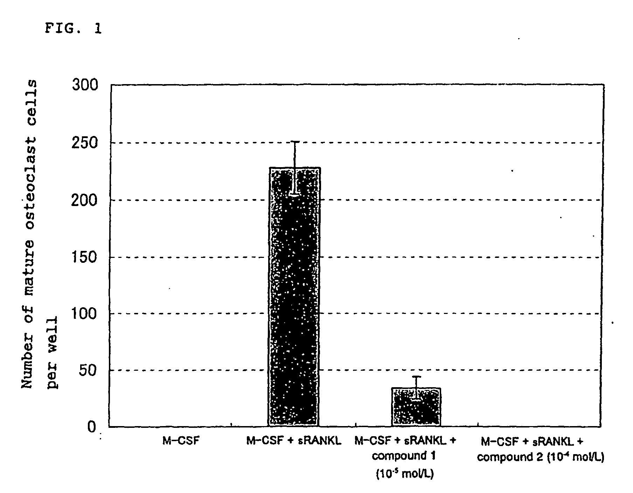

Fig. 1 is a graph showing the mean and SD values for an experiment in which the number of mature osteoclasts that were produced per well in 48-well plates in accordance with the procedure described in Test 1 was evaluated for n = 3.

### MODES FOR CARRYING OUT THE INVENTION

Examples of the halogen atom in the linear or branched halogenoalkyl group as R₁ which has 1-7 carbon atoms include fluorine, chlorine, bromine and iodine, with fluorine being particularly preferred. At least one halogen atom needs to be present. If two or more halogen atoms are present, they may be the same or different; preferably, they are the same to make a perhalogenoalkyl.

The alkyl group in the linear or branched halogenoalkyl group as R₁ which has 1-7 carbon atoms is preferably a linear or branched alkyl group having 1-5 carbon atoms, with linear or branched alkyl groups of 1-4 carbon atoms, i.e. methyl, ethyl, n-propyl, i-propyl, n-butyl, i-butyl, sec-butyl and tert-butyl, being more preferred, among which the n-butyl group being particularly preferred. Examples of the linear or branched alkyl group having 1-5 carbon atoms in the invention include methyl, ethyl, n-propyl, i-propyl, n-butyl, i-butyl, sec-butyl, tert-butyl, n-pentyl, 1-methylbutyl, 2-methylbutyl, 3-methylbutyl, 1,1-dimethylpropyl, 1,2-dimethylpropyl, 2,2-dimethylpropyl, 1-ethylpropyl, etc.

The linear or branched halogenoalkyl group as R₁ which has 1-7 carbon atoms is preferably a linear or branched perhalogenoalkyl group having 1-5 carbon atoms and a linear or branched perfluoroalkyl group having 1-5 carbon atoms is more preferred, with specific examples including trifluoromethyl, pentafluoroethyl, heptafluoro-n-propyl, heptafluoro-i-propyl, nonafluoro-n-butyl, nonafluoro-i-butyl, nonafluoro-sec-butyl, nonafluoro-tert-butyl, undecafluoro-n-pentyl, octafluoro-1-trifluoromethyl-butyl, octafluoro-2-trifluoromethyl-butyl, octafluoro-3-trifluoromethyl-butyl, pentafluoro-1,1-bis-trifluoromethyl-propyl, pentafluoro-1,2-bis-trifluoromethyl-propyl, pentafluoro-2,2-bis-trifluoromethyl-propyl, hexafluoro-1-pentafluoroethylpropyl, etc. Particularly preferred is the nonafluoro-n-butyl group.

When Ra is a hydroxyl group, specific examples of the linear or branched alkynyl group as Rb which has 2-5 carbon atoms include an ethynyl group, a 1-propynyl group, a 1-butynyl group, a 1-n-pentynyl group, a 3-methyl-1-butynyl group, etc. Alternatively, Ra and Rb, when taken together with the carbon atom to which they are bound, represent a carbonyl group (-(C=O)-). In a specific example of the combination of Ra and Rb, a hydroxyl group as Ra may be combined with an ethynyl group as Rb.

In the present invention, m is preferably an integer selected from among 4, 5, 6, 7, 8, 9 and 10, with 8 being more preferred.

In the present invention, n is preferably an integer selected from among 2, 3, 4, 5 and 6, with 3 being more preferred.

The compounds of the invention can exist as stereoisomers and such stereoisomers, either individually or in admixture, are all included within the present invention. The stereoisomers in the invention embrace geometric isomers, optical isomers and diastereomers. As regards the asymmetric center on the steroidal nucleus represented by formula (I), it is particularly preferred that the group represented by formula (II) binds to a 7α- or an 11β-position. When Ra is a hydroxyl group, it preferably binds to a 17β-position whereas Rb binds to a 17α-position. Compounds having R- or S-configuration at the carbon to which a carboxylic acid or a carboxylic acid salt or ester is bonded, wherein said carbon is the carbon in the group of formula (II) are preferable.

The compounds of the invention may also be obtained as hydrates.

In order to obtain stereoisomers of the compounds of the invention as single entities, mixtures of stereoisomers may be resolved by using a chiral column. The method of using a chiral column may be performed using CHIRAL PAK-OT(+), OP(+), AD, CHIRAL CEL-OA, OB, OJ, OK, OC, OD, OF and OG (all being the trade names of DAICEL CHEMICAL INDUSTRIES, LTD.), etc. Using single stereoisomers as starting materials, one can obtain stereoisomers of the corresponding compounds of the invention as single entities.

The compounds of the invention can also be obtained as salts which may be exemplified by salts of alkali metals such as sodium and potassium, salts of alkaline earth metals such as magnesium and calcium, salts of rare earth metals such as cerium and samarium, and salts of other metals such as zinc and tin. Pharmaceutically acceptable salts are preferred in the present invention and examples include alkali metal salts such as sodium and potassium salts, and alkaline earth metal salts such as calcium and magnesium salts.

The compounds of the invention may also be obtained as esters and examples include the following: lower alkyl esters such as methyl ester, ethyl ester and t-butyl ester; substituted lower alkyl esters such as methoxymethyl ester, methylthiomethyl ester, tetrahydropyranyl ester, methoxyethoxymethyl ester, benzyloxymethyl ester, phenacyl ester, diacylmethyl ester, phthalimidomethyl ester, 2,2,2-trichloroethyl ester, 2-chloroethyl ester, 2-(trimethylsilyl)ethyl ester, 2-methylthioethyl ester and 2-(p-toluenesulfonyl)ethyl ester; substituted benzyl esters such as benzyl ester, diphenylmethyl ester, triphenylmethyl ester, p-nitrobenzyl ester, p-methoxybenzyl ester and 2-(9,10-dioxo)anthrylmethyl ester; and silyl esters such as trimethylsilyl ester, t-butyldimethylsilyl ester and phenyldimethylsilyl ester.

The compounds within the scope of the invention can be formulated as pharmaceutical compositions containing one or more pharmaceutically acceptable additives such as diluents, moistening agents, emulsifiers, dispersing agents, promoters, antiseptics, buffers, binders and stabilizers and may be administered in any suitable form that depends on the intended route of administration which may be parenteral or oral.

The compounds of the invention may be administered at doses that can be chosen as appropriate for various factors including the physique of the patient, his or her age, physical condition, the severity of the disease and the elapsed time after its manifestation. Because the compound of the invention is expected to show a significantly high activity by oral route, the compounds are generally used in doses of 0.1-500 mg/day/person; in the case of parenteral administration (intravenous, intramuscular or subcutaneous), they are generally used in doses of 0.1-1000 mg/day/person or 0.1-1000 mg/month/person.

The compounds of formula (I) can be produced by either one of the methods depicted in reaction schemes A and B set forth below. In reaction schemes A and B, symbols n, m and R₁ have the same meanings as defined in formula (II).

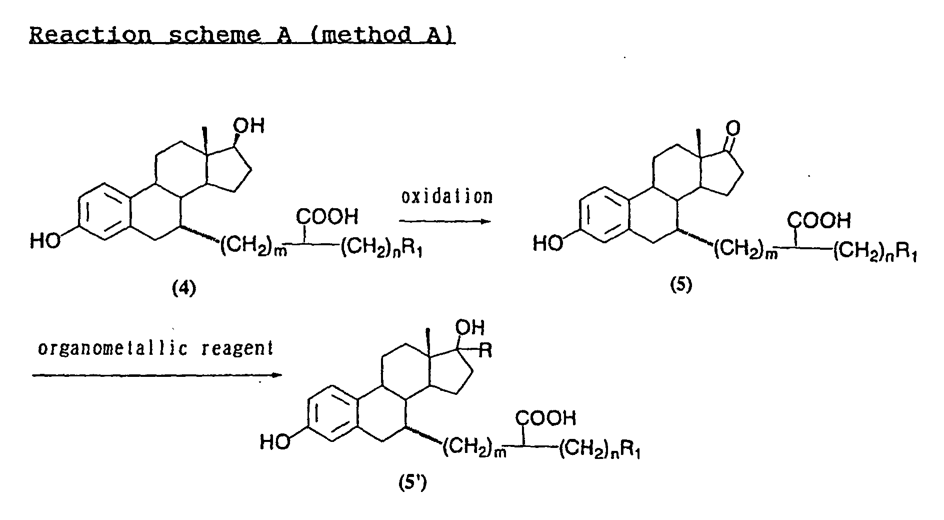

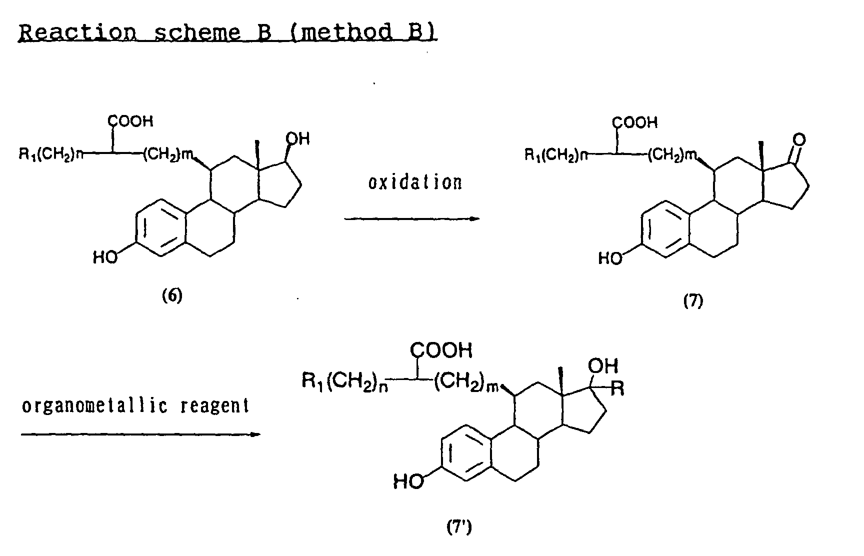

### (Method A)

The compound of formula (5) can be synthesized by the following procedure. With the compound of formula (4) used as a starting material, the hydroxyl group at 17-position is oxidized by Oppenauer oxidation, Jones oxidation, PCC oxidation, Swern oxidation or oxidation with ruthenium (e.g. TPAP) so as to synthesize the compound of formula (5).

The compound of formula (5) is then reacted with an organometallic reagent (e.g. RLi, RNa, RK, RMgX, RR'R"Al or RR'Zn, where R is a linear or branched alkynyl group having 2-5 carbon atoms, R' and R" is a linear or branched alkyl group having 2-5 carbon atoms, a linear or branched alkenyl group having 2-5 carbon atoms or a linear or branched alkynyl group having 2-5 carbon atoms) in a solvent inert to the reaction of interest (e.g. dimethyl sulfoxide, ether solvents such as tetrahydrofuran, dimethoxyethane, dioxane and diethyl ether, or dimethylformamide, toluene or dichloromethane) at a temperataure between -78°C and the boiling point of the reaction mixture, preferably between -78°C and room temperature, thereby producing the compound of formula (5').

If oxidation is performed by Oppenauer oxidation, the reaction may be carried out in the presence of a metallic reagent and an oxidizer that are commonly employed in Oppenauer oxidation. Examples of the metallic reagent that can be used include aluminum alkoxides, zirconium alkoxide and ruthenium reagents. Preferred aluminum alkoxides include aluminum triisopropoxide, aluminum tri-tert-butoxide, aluminum triphenoxide and aluminum triethoxide, with aluminum triisopropoxide and aluminum tri-tert-butoxide being more preferred. Preferred zirconium alkoxides include zirconium ethoxide, zirconium propoxide, zirconium isopropoxide and zirconium tert-butoxide, with zirconium tert-butoxide being more preferred. Ruthenium reagents are preferably obtained from commercial sources, with ruthenium dichloride tris-triphenylphosphine being particularly preferred. The metallic reagents are preferably used in amounts of 0.1-2 equivalents, more preferably 0.5-1.1 equivalents. As the oxidizer, ketones and aldehydes which are commonly used in Oppenauer oxidation may be employed and preferred examples are acetone, cyclohexanone, acetophenone, p-benzoquinone, benzaldehyde optionally having a substituent such as a nitro group and chloral, with cyclohexanone and a benzaldehyde being more preferred. The oxidizer is preferably used in amounts of 1-100 equivalents, more preferably 5-10 equivalents. The reaction solvent may be chosen from commonly used solvents that are inert to the reaction and preferred examples include benzene, toluene, xylene and dichloromethane, with toluene being particularly preferred. The concentration in the reaction solvent is preferably in the range of 0.01-2.0 M and considering the reaction rate and yield, as well as the isomerization of the asymmetric center, the range of 0.05-0.1 M is more preferred. The reaction temperature is preferably in the range of 0-200°C, more preferably in the range of 25-120°C.

In method A, by using a single stereoisomer of the compound of formula (4) as the starting material, one can produce a single stereoisomer of the corresponding compound of formula (5).

The compound of formula (4) for use as the starting material can be synthesized by the method described in WO 01/42186. A single isomer of the compound of formula (4) can also be obtained by the method described in WO 01/42186.

### (Method B)

The compound of formula (7) can be synthesized from the compound of formula (6) by the same procedure as in method A. The compound of formula (7') can be synthesized from the compound of formula (7) by the same procedure as in method A. The compound of formula (6) as the starting material can be synthesized by the method described in WO 01/42186.

### Examples

The following examples are provided for further illustrating the present invention but are in no way to be taken as limiting.

### Example 1

### [Step 1] Optical resolution of 10-(3,17β-dihydroxyestra-1,3,5(10)-trien-7α-yl)-2-(4,4,5,5,6,6,7,7,7-nonafluoroheptyl)decanoic acid

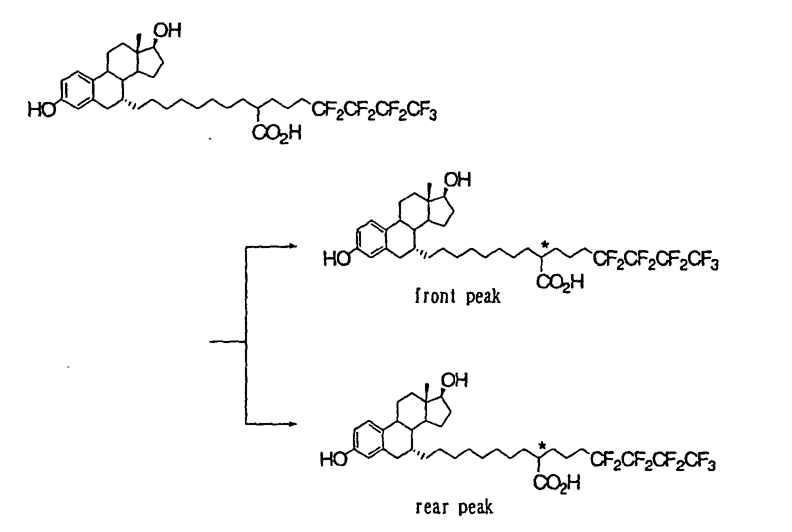

A starting material, 10-(3,17β-dihydroxyestra-1,3,5(10)-trien-7α-yl)-2-(4,4,5,5,6,6,7,7,7-nonafluoroheptyl)decanoic acid, was prepared in accordance with the method described in WO 01/42186.

This compound (138 g) was optically resolved by a chiral column (CHIRAL PACK AD of DAICEL CHEMICAL INDUSTRIES, LTD.) using a 90:10:0.1 mixture of hexane, isopropanol and acetic acid as a mobile phase, producing 58.3 g of the first component (front peak) and 58.8 g of the second component (rear peak).

### [Step 2]

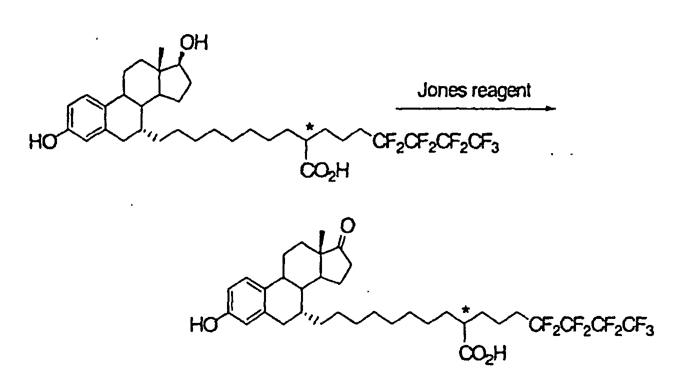

A portion (2.71 g, 3.86 mmol) of the second component obtained in step 1 was dissolved in acetone (40 ml) and a separately prepared Jones reagent was slowly added dropwise at -10°C until the reaction solution turned brown. After confirming the end of the reaction by thin-layer chromatography (TLC), the mixture was further stirred for 30 minutes and isopropyl alcohol was added to the mixture. After adding water, the mixture was extracted with ethyl acetate and the organic layer was washed with brine, followed by drying over anhydrous sodium sulfate. The organic solvent was concentrated under vacuum and the residue was purified by silica gel column chromatography (ethyl acetate/hexane = 1/2) to yield 10-(3-hydroxy-17-oxo-estra-1,3,5(10)-trien-7α-yl)-2-(4,4,5,5,6,6,7,7,7-nonafluoroheptyl)decanoic acid (compound 1) in an amount of 1.51 g (56%).
¹H-NMR(270MHz, CDCl₃): δ 7.14 (d, J=8.4Hz, 1H, C1-CH), 6.64 (dd, J=2.4, 8.4Hz, 1H, C2-CH), 6.56 (d, J =2.4Hz, 1H, C4-CH), 2.89 (dd, J=16.0, 3.4Hz, 1H), 2.73 (d, J=16.0Hz, 1H), 2.58-2.30 (m, 4H), 2.23-1.80 (m, 8H), 1.80-1.00 (m, 25H), 0.91 (s, 3H).

### Example 2

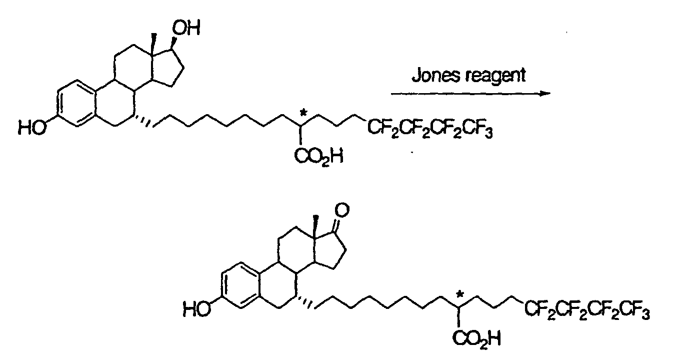

A portion (2.78 g, 3.96 mmol) of the first component obtained in step 1 of Example 1 was dissolved in acetone (40 ml) and a separately prepared Jones reagent was slowly added dropwise at -10°C until the reaction solution turned brown. After confirming the end of the reaction by TLC, the mixture was further stirred for 30 minutes and isopropyl alcohol was added to the mixture. After adding water, the mixture was extracted with ethyl acetate and the organic layer was washed with brine, followed by drying over anhydrous sodium sulfate. The organic solvent was concentrated under vacuum and the residue was purified by silica gel column chromatography (ethyl acetate/hexane = 1/2) to yield 10-(3-hydroxy-17-oxo-estra-1,3,5(10)-trien-7α-yl)-2-(4,4,5,5,6,6,7,7,7-nonafluoroheptyl)decanoic acid (compound 2) in an amount of 1.55 g (56%).
¹H-NMR(270MHz, CDCl₃): δ 7.14 (d, J=8.4Hz, 1H, C1-CH), 6.64 (dd, J=2.4, 8.4Hz, 1H, C2-CH), 6.56 (d, J =2.4Hz, 1H, C4-CH), 2.89 (dd, J=16.0, 3.4Hz, 1H), 2.73 (d, J=16.0Hz, 1H), 2.58-2.30 (m, 4H), 2.23-1.80 (m, 8H), 1.80-1.00 (m, 25H), 0.91 (s, 3H).

### Example 3

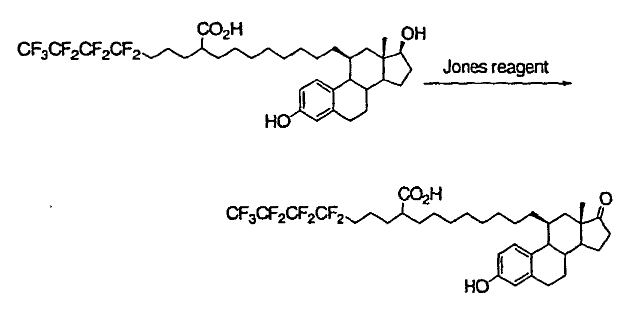

An amount (57 mg, 0.08 mmol) of 10-(3,17β-dihydroxyestra-1,3,5(10)-trien-11β-yl)2-(4,4,5,5,6,6,7,7,7-nonafluoroheptyl)decanoic acid was dissolved in acetone (1.0 ml) and a separately prepared Jones reagent was slowly added dropwise at -10°C until the reaction solution turned brown. After confirming the end of the reaction by TLC, the mixture was further stirred for 30 minutes and isopropyl alcohol was added to the mixture. After adding water, the mixture was extracted with ethyl acetate and the organic layer was washed with brine, followed by drying over anhydrous sodium sulfate. The organic solvent was concentrated under vacuum and the residue was purified by silica gel column chromatography (ethyl acetate/hexane = 1/2) to yield 10-(3-hydroxy-17-oxo-estra-1,3,5(10)-trien-11β-yl)-2-(4,4,5,5,6,6,7,7,7-nonafluoroheptyl)decanoic acid (compound 3) in an amount of 32 mg (56%).
¹H-NMR(270MHz, CDCl₃): δ 7.00 (d, J=8.4Hz, 1H, C1-CH), 6.64 (dd, J=2.4, 8.4Hz, 1H, C2-CH), 6.56 (d, J =2.4Hz, 1H, C4-CH), 2.81-2.65 (m, 2H), 2.61-2.30 (m, 5H), 2.20-1.92 (m, 8H), 1.80-1.00 (m, 24H), 1.03 (s, 3H).

### Example 4

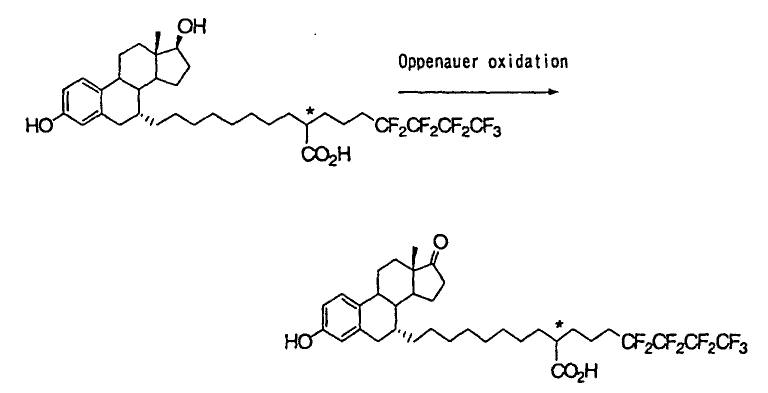

A portion (2.71 g, 3.86 mmol) of the second component obtained in accordance with the procedure of step 1 in Example 1 and cyclohexanone (3.78 g, 38.6 mmol) were dissolved in toluene (40 ml) and aluminum tri-tert-butoxide (1.05 g, 4.25 mmol) was added to the solution at room temperature. The reaction mixture was heated to 100°C under nitrogen atmosphere, stirred for 2 hours and then cooled to room temperature. 1 N aqueous solution of hydrochloric acid and ethyl acetate were added to the reaction mixture, which was stirred vigorously and then left to stand for 24 hours. The organic layer was extracted, washed with brine and dried over anhydrous sodium sulfate. The organic solvent was concentrated under vacuum and the residue was purified by silica gel column chromatography (acetone/hexane = 1/2) to yield 10-(3-hydroxy-17-oxo-estra-1,3,5(10)-trien-7α-yl)-2-(4,4,5,5,6,6,7,7,7-nonafluoroheptyl)decanoic acid in an amount of 1.51 g (89%).
¹H-NMR(270MHz, CDCl₃): δ 7.14 (d, J=8.4Hz, 1H, C1-CH), 6.64 (dd, J=2.4, 8.4Hz, 1H, C2-CH), 6.56 (d, J =2.4Hz, 1H, C4-CH), 2.89 (dd, J=16.0, 3.4Hz, 1H), 2.73 (d, J=16.0Hz, 1H), 2.58-2.30 (m, 4H), 2.23-1.80 (m, 8H), 1.80-1.00 (m, 25H), 0.91 (s, 3H).

### Example 5

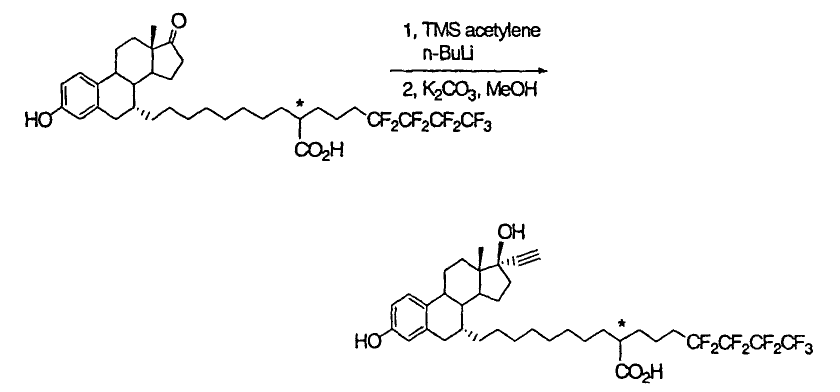

Trimethylsilyl acetylene (3.9 ml, 27.8 mmol) was dissolved in dehydrated tetrahydrofuran (27 ml) and the solution was cooled to -78°C under nitrogen atmosphere. A solution of n-butyllithium in hexane (17.14 ml, 27.16 mmol) was slowly added dropwise with the temperature held at -78°C and the mixture was stirred for 10 minutes at that temperature. Subsequently, compound 1 (3.9 g, 5.54 mmol) obtained in Example 4 was dissolved in tetrahydrofuran (27 ml) and the solution was added to the previously prepared reaction mixture at -78°C. Following 10 minutes stirring, the mixture was warmed to room temperature and stirred for an additional hour and a half. The mixture was again cooled to 0°C and the reaction was quenched by adding methanol to the reaction mixture. Subsequently, potassium carbonate (4.0 g) was added and the mixture was stirred for 3 hours at room temperature. Dilute hydrochloric acid (pH -1) was added and the organic layer was extracted with ethyl acetate, washed with brine and dried over anhydrous sodium sulfate. The organic solvent was concentrated under vacuum and the residue was purified by silica gel column chromatography (acetone/hexane = 1/4 - 1/2), then by octadecyl column chromatography (RP-18 column; acetonitrile/water = 75/25) using a medium-pressure analytical column purifier to yield 10-(3,17β-dihydroxy-17α-ethynyl-estra-1,3,5(10)-trien-7α-yl)-2-(4,4,5,5,6,6,7,7,7-nonafluoroheptyl)decanoic acid (compound 4) in an amount of 2.86 g (71%).
¹H-NMR(270MHz, CDCl₃) : δ 7.14 (d, J=8.4Hz, 1H, C1-CH), 6.63 (dd, J=2.5, 8.4Hz, 1H, C2-CH), 6.54 (d, J =2.5Hz, 1H, C4-CH), 2.86 (dd, J=16.2, 5.0Hz, 1H), 2.70 (d, J=16.2Hz, 1H), 2.62 (s, 1H, acetylene-CH), 2.45-2.25 (m, 4H), 2.18-1.80 (m, 8H), 1.80-0.90 (m, 25H), 0.89 (s, 3H).
LC-MASS : m/e 727(M⁺+1)
HPLC (λ=280 nm) : >99% (Chiralpack AD) (Hexane:i-PrOH:AcOH=90:10:0.1, 1.0 ml/min, 30°C) >99% (YMC-ODS-A)(MeCN:H₂O:TFA=75:25:0.1, 1.0 ml/min, 25°C)

### Example 6

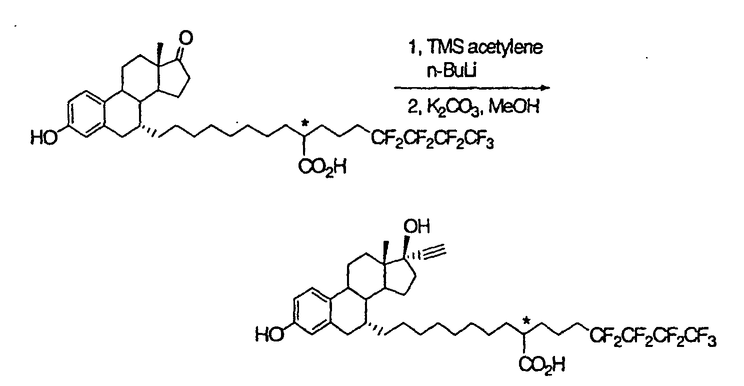

Trimethylsilyl acetylene (1.82 ml, 13.1 mmol) was dissolved in dehydrated tetrahydrofuran (6.4 ml) and the solution was cooled to -78°C under nitrogen atmosphere. A solution of n-butyllithium in hexane (8.0 ml, 12.5 mmol) was slowly added dropwise with the temperature held at -78°C and the mixture was stirred for 10 minutes at that temperature. Subsequently, a portion (920 mg, 1.31 mmol) of compound 2 obtained in Example 2 was dissolved in tetrahydrofuran (13.8 ml) and the solution was added to the previously prepared reaction mixture at -78°C. Following 10 minutes stirring, the mixture was warmed to room temperature and stirred for an additional hour and a half, followed by adding methanol (18.4 ml) to the reaction mixture. Subsequently, potassium carbonate (920 mg) was added and the mixture was stirred for 1.5 hours at room temperature. Dilute hydrochloric acid (pH ∼2) was added and the organic layer was extracted with ethyl acetate and dried over anhydrous sodium sulfate. The organic solvent was concentrated under vacuum and the residue was purified by silica gel column chromatography (acetone/hexane = 1/3 - 1/2) and only the fractions of high purity were collected to yield 10-(3,17β-dihydroxy-17α-ethynyl-estra-1,3,5(10)-trien-7α-yl)-2-(4,4,5,5,6,6,7,7,7-nonafluoroheptyl)decanoic acid (compound 5) in an amount of 489 mg (51%).
¹H-NMR(270MHz, CDCl₃): δ 7.15 (d, J=8.4Hz, 1H, C1-CH), 6.63 (dd, J=2.7, 8.4Hz, 1H, C2-CH), 6.54 (d, J =2.7Hz, 1H, C4-CH), 2.86 (dd, J=16.2, 5.1Hz, 1H), 2.71 (d, J=16.2Hz, 1H), 2.62 (s, 1H, acetylene-CH), 2.45-2.25 (m, 4H), 2.18-1.80 (m, 8H), 1.80-0.90 (m, 25H), 0.89 (s, 3H).
HPLC (λ=280 nm) : >99% (Chiralpack AD)(Hexane:i-PrOH:AcOH=90:10:0.1, 1.0 ml/min, 25°C) >98% (YMC-ODS-A)(MeCN:H₂O:TFA=75:25:0.1, 1.0 ml/min, 25°C)

### [Test 1] Effects on the Process of Differentiation of Bone Marrow Derived Osteoclast Precursor Cells into Mature Osteoclasts

Bone marrow cells separated from the vertebrae of *ddY* mice (male, 7-9 week old) were cultivated on cell culture plates for 3 days in the presence of a macrophage colony-stimulating factor (M-CSF, 10 ng/ml). The suspended cells were washed with PBS and removed whereas the cells adhering to the plates were used as osteoclast precursor cells. Following the addition of more M-CSF (10 ng/ml) and a soluble RANK ligand (sRANKL, 40 ng/ml), the precursors were cultivated for 3-4 days in order to induce the maturation of osteoclasts. Before this cultivation, either compound 1 or a solvent (ethanol) alone was added in order to see what effects they would have. The maturation of osteoclasts was evaluated by staining the cultivated cells with a tartrate resistant acid phosphatase (TRAP) and counting the number of trinuclear and higher TRAP stained cells. Table 1 and Fig. 1 show the mean and SD values for the experiment in which the number of mature osteoclasts that were produced per well in 48-well plates was evaluated for n = 3.

**Table 1**

| Stimulant | No. of mature osteoclasts per well |
|---|---|
| M-CSF | 0 ±0 |
| M-CSF + sRANKL | 228 ±23 |
| M-CSF + sRANKL + Compound 1(10⁻⁵mol/L) | 34 ±10 |
| M-CSF + sRANKL + Compound 1(10⁻⁴mol/L) | 0 ±0 |

### [AUC measurement in oral administration]

The AUC of specified compounds in oral administration was measured by the following tests.

### [Test 2] Test with mice

A 5% gum arabic suspension (10 ml/kg) of a test substance (30 mg/kg) was forcibly administered orally into ICR mice (female, 8-10 week old) by means of a mouse stomach probe and after the lapse of specified periods of time (15 minutes, 30 minutes, and 1, 2, 4, 6, 8, 24 and 48 hours), whole blood was taken from the abdominal large vein (n=3 for each time period). The concentration of the test substance in the sampled plasma was measured by HPLC (eluant: acetonitrile/water) and AUC was determined by moment analysis from the average plasma concentration for each time period. The test substances were compound 1, compound 2, compound 4 and compound 5. Two control compounds were used, one being the first component prepared in step 1 of Example 1 (control compound 2) and the other being the second component (control compound 1).

**Table 2**

| Test substance | AUC_{∞}(µg·h/ml) |
|---|---|
| Compound 1 | 468.35 |
| Compound 2 | 722.55 |
| Compound 4 | 202.10 |
| Compound 5 | 661.00 |
| Control compound 1 | 273.64 |
| Control compound 2 | 469.73 |

Comparing with control compounds 1 and 2 having hydroxyl group at 17-position, the compounds of the present invention had high AUC in oral administration and are expected to show potent pharmacological efficacy in oral administration.

### [Test 3] Test with monkeys

A PEG/water/ethanol [8/4/3 (v/v)] solution (3 ml/kg) of a test substance (3 mg/kg) was administered orally into cynomolgus monkeys (female, 3-5 years old, 2.5-4 kg) through a catheter inserted from the nostrils into the stomach and about 1 ml of blood was taken over a period of 48 hours at specified time intervals (15 minutes, 30 minutes, and 1, 2, 4, 6, 8, 10, 24 and 48 hours). The concentration of the test substance in the sampled plasma was measured by use of LC/MS/MS (ESI, eluant: acetonitrile/water) and AUC was determined by moment analysis. The test substances were compound 1 and compound 4. The second component prepared in step 1 of Example 1 was used as control compound 1.

**Table 3**

| Test substance | AUC_{∞}(µg·h/ml) |
|---|---|
| Compound 1 | 2.42 |
| Compound 4 | 3.8 |
| Control compound 1 | 0.42 |

Comparing with control compound 1 having hydroxyl group at 17-position, the compounds of the present invention had high AUC in primate monkeys and are expected to show potent pharmacological efficacy when administered orally to primates including humans. Primates are all animals of the order Primates including humans.

### [Test 4] Anti-estrogen activity (in oral administration)

For anti-estrogen activity measurement, mice (ICR, body weight = 30 ± 2 g) that had received ovariectomy 2 weeks before were injected subcutaneously for 3 days with 17β-estradiol benzoate (Sigma) in doses of 0.1 µg per animal and the degree by which a test compound suppressed the increase in the weight of the uterus was measured. In the test, each of the test compounds and a control compound were suspended in a 5% gum arabic solution and administered to the mice orally in doses of 10 mg/kg on a once-a-day basis for 3 days. Twenty-four hours after the administration of the final dose, the test animals were slaughtered and the uterus was extracted and its weight was measured. The results of the measurement are shown in Table 4 below.

**Table 4**

| Test compound | Percent suppression (%) |
|---|---|
| Compound 1 | 96 |
| Compound 3 | 99 |
| Compound 4 | 94 |
| Compound 5 | 96 |

### [Test 5] Estrogen Receptor Downregulating Activity

### Reagents and equipment

Tris(hydroxymethyl)aminomethane, dithiothreitol and glycerol were obtained from Nacalai Tesque. Protease inhibitor tablets were obtained from Boehringer Mannheim. Human breast cancer cell line MCF-7 was obtained from ATCC, fetal calf serum (FCS) from Hyclone, DMEM from Invitrogen, and PBS(-) from Nissui.

### Preparing a solubilizing buffer

Tris-HCl buffer (1 mol/L, pH = 7.4) was diluted 50-fold with distilled water to a concentration of 20 mmol/L (pH = 7.4). To the diluted buffer, dithiothreitol and glycerol were added to give respective final concentrations of 1 mmol/L and 10%. Further, protease inhibitor tablets were added, with one tablet per 50 mL of buffer. The thus conditioned solution was used as a solubilizing buffer.

### Quantitation of intranuclear estrogen receptor (EgR)

Samples for quantitating the intranuclear estrogen receptor (EgR) were prepared by the following procedure. MCF-7 cultivated in 5% FCS/DMEM was sown on 6-cm dishes at a concentration of 4 x 10⁵ cells/dish and cultivated for 3 days; cultivation was continued for an additional 2 days in the presence of compound 1 or compound 2 at final concentrations of 1-100 nmol/L or in the presence of compound 4 at final concentrations of 1-1,000 nmol/L or compound 5 at final concentrations of 1-10,000 nmol/L. Thereafter, the cells were washed with PBS(-), mixed with the solubilizing buffer, subjected to two cycles of freezing at -80°C and thawing at room temperature, and centrifuged at 4°C for 10 minutes at 4000 rpm (centrifuge: TOMY, Model MX-150). To the precipitation, the solubilizing buffer containing 500 mmol/L of NaCl was added and the mixture was treated with a micro-tube mixer (TOMY, Model MT-360) at 4°C for about 30 minutes. Following centrifugation at 4°C for 30 minutes at 15,000 rpm (centrifuge: TOMY, Model MX-150), the supernatant was collected and stored at -80°C as an extracted nuclear sample. The EgR content in the extracted nuclear sample was measured with an EgR assay kit (ER-EIA "ABBOT" of Dynapot).

As test results, IC₅₀ values for compounds 1, 2, 4 and 5 are listed in Table 5. Obviously, compounds 1, 2, 4 and 5 reduced the intranuclear EgR content in a dose-dependent manner.

**Table 5**

| | IC₅₀(nmol/L) |
|---|---|
| Compound 1 | 8.4 |
| Compound 2 | 8.7 |
| Compound 4 | 14 |
| Compound 5 | 100 |

Faslodex, an estrogen receptor downregulator, has been shown in clinical tests to be superior to a SERM drug and an aromatase inhibitor in terms of objective response rate and duration of response to breast cancer (Journal of Clinical Oncology, 20, 3386-3395, 2002; Endocrine-Related Cancer 9, 267-276, 2002) and to be also effective against tamoxifen-resistant breast cancer (British Journal of Cancer 74, 300-308, 1996).

As is clear from Test 5, the compounds of the invention have an estrogen receptor downregulating activity, so they are expected to be more potent therapeutics of breast cancer than SERM drugs and aromatase inhibitors, as well as proving more effective against tamoxifen-resistant breast cancer.

### INDUSTRIAL APPLICABILITY

Having outstanding pharmacological activities such as the ability to suppress the differentiation of osteoclast precursor cells into osteoclasts, anti-estrogen activity and the activity in downregulating the estrogen receptor, the compounds of the invention are useful as medicines. They also exhibit outstanding pharmacological effects such as anti-estrogen activity in oral administration and, hence, are useful as medicines. Further, the compounds of the invention have high AUC in oral administration and are useful as medicines for oral administration. The compounds of the invention show particularly high AUC when administered to primates, so it is particularly preferred to apply them as medicines for primates including humans.

## Claims

1. A compound of formula (I) 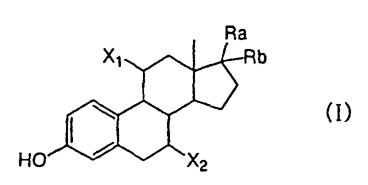 (where X₁ and X₂ represent independently a hydrogen atom or a group of formula (II) 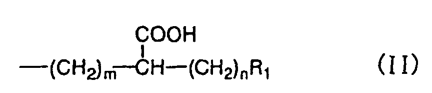 R₁ represents a linear or branched halogenoalkyl group having 1-7 carbon atoms;
Ra represents a hydroxyl group and Rb represents a linear or branched alkynyl group having 2-5 carbon atoms, or Ra and Rb, when taken together with the carbon to which they are bound, represent a carbonyl group;
m is an integer of 2-14;
n is an integer of 2-7;
provided that X₁ and X₂ are not both a hydrogen atom), stereoisomers of the compound, or hydrates, salts or esters thereof.

2. A compound of formula (Ia) 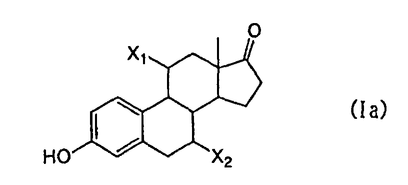 (where X₁ and X₂ represent independently a hydrogen atom or a group of formula (II) 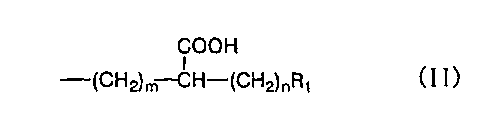 R₁ represents a linear or branched halogenoalkyl group having 1-7 carbon atoms;
m is an integer of 2-14;
n is an integer of 2-7;
provided that X₁ and X₂ are not both a hydrogen atom), stereoisomers of the compound, or hydrates, salts or esters thereof.

3. A compound of formula (Ib) 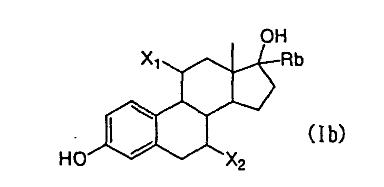 (where X₁ and X₂ represent independently a hydrogen atom or a group of formula (II) 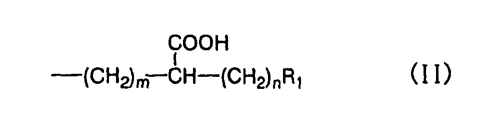 R₁ represents a linear or branched halogenoalkyl group having 1-7 carbon atoms;
Rb represents a linear or branched alkynyl group having 2-5 carbon atoms;
m is an integer of 2-14;
n is an integer of 2-7;
provided that X₁ and X₂ are not both a hydrogen atom), stereoisomers of the compound, or hydrates, salts or esters thereof.

4. The compound, stereoisomers of the compound, or hydrates, salts or esters thereof according to any one of claims 1-3, wherein m is an integer of 4-10 and n is an integer of 2-6.

5. The compound, stereoisomers of the compound, or hydrates, salts or esters thereof according to any one of claims 1-3, wherein m is 8 and n is 3.

6. A pharmaceutical composition comprising the compound, stereoisomers of the compound, or hydrates, salts or esters thereof according to any one of claims 1-3 as an active ingredient.

7. The pharmaceutical composition according to claim 6 which is used to prevent or treat osteoporosis.

8. The pharmaceutical composition according to claim 6 which is used to prevent or treat breast cancer.

9. A process for producing a compound of formula (Ia) 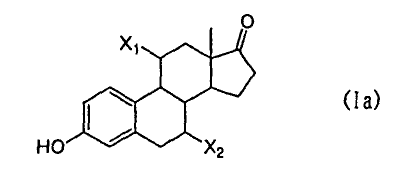 (where X₁ and X₂ represent independently a hydrogen atom or a group of formula (II) 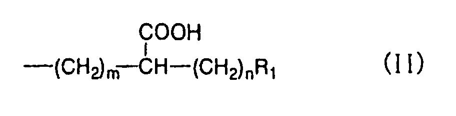 R₁ represents a linear or branched halogenoalkyl group having 1-7 carbon atoms;
m is an integer of 2-14;
n is an integer of 2-7;
provided that X₁ and X₂ are not both a hydrogen atom), stereoisomers of the compound, or hydrates, salts or esters thereof, said process including the step of oxidizing a compound of formula (III) 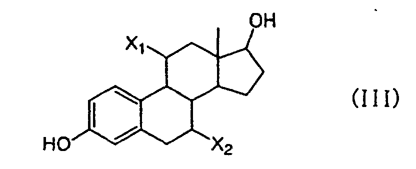 (where X₁ and X₂ represent independently a hydrogen atom or a group of formula (II) 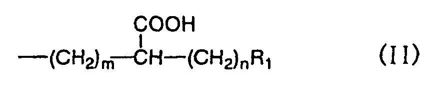 R₁ represents a linear or branched halogenoalkyl group having 1-7 carbon atoms;
m is an integer of 2-14;
n is an integer of 2-7;
provided that X₁ and X₂ are not both a hydrogen atom), stereoisomers of the compound, or hydrates, salts or esters thereof.

10. The process according to claim 9, in which the oxidation reaction is performed by Oppenauer oxidation.

11. A process for producing a compound of formula (Ib) 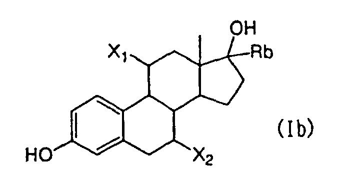 (where X₁ and X₂ represent independently a hydrogen atom or a group of formula (II) 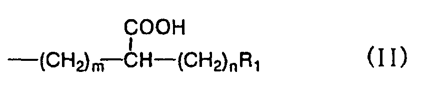 R₁ represents a linear or branched halogenoalkyl group having 1-7 carbon atoms;
Rb represents a linear or branched alkynyl group having 2-5 carbon atoms;
m is an integer of 2-14;
n is an integer of 2-7;
provided that X₁ and X₂ are not both a hydrogen atom), stereoisomers of the compound, or hydrates, salts or esters thereof, said process including the step of alkynylating a compound of formula (Ia) 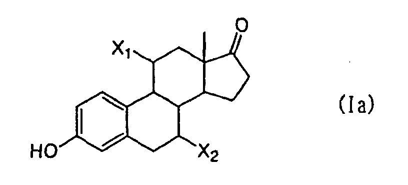 (where X₁ and X₂ represent independently a hydrogen atom or a group of formula (II) 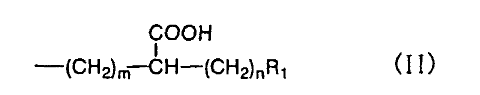 R₁ represents a linear or branched halogenoalkyl group having 1-7 carbon atoms;
m is an integer of 2-14;
n is an integer of 2-7;
provided that X₁ and X₂ are not both a hydrogen atom), stereoisomers of the compound, or hydrates, salts or esters thereof.
